# EUROPEAN PATENT APPLICATION

(11) **EP 4 318 497 A1**
(43) Date of publication of application: **07.02.2024**
(21) Application number: 22796033.3
(22) Date of filing: 20.04.2022
(51) Int. Cl.: G16H 50/70, G16H 30/40, G16H 50/20, G06N 3/04, G06N 3/08, A61B 10/00

(54) **TRAINING METHOD FOR TRAINING ARTIFICIAL NEURAL NETWORK FOR DETERMINING BREAST CANCER LESION AREA, AND COMPUTING SYSTEM PERFORMING SAME**

(30) Priority: 28.04.2021 KR 20210055207
(71) Applicant: Deep Bio Inc., Seoul 08380 (KR)
(72) Inventor: CHOI, Jun Young, Seoul 04428 (KR); KWAK, Tae Yeong, Seoul 05119 (KR); KIM, Sun Woo, Seongnam-si, Gyeonggi-do 13599 (KR)
(74) Representative: Flügel Preissner Schober Seidel
(86) International application number: PCT/KR2022/005634
(87) International publication number: WO 2022/231200

(57) **Abstract**

Disclosed are a training method for training an artificial neural network capable of determining a breast cancer lesion area in consideration of both microscopic features and macroscopic features of biological tissue, and a computing system for performing same. According to one aspect of the present invention, provided is a method for training an artificial neural network, comprising steps in which: an artificial neural network training system acquires a slide image of a biological tissue slide; the artificial neural network training system acquires, from the slide image, a first high-resolution patch to an Nth high-resolution patch; the artificial neural network training system acquires an ith low-resolution patch corresponding to an ith high-resolution patch (1<=i<=N); and the artificial neural network training system inputs the ith high-resolution patch and the ith low-resolution patch to train the artificial neural network.

## Description

### Technical Field

The present disclosure relates to a training method for training an artificial neural network for determining a breast cancer lesion area, and a computing system performing the same. More specifically, the present disclosure relates to a training method for training an artificial neural network for determining a breast cancer lesion area by considering both microscopic and macroscopic characteristics of biological tissue, and a computing system performing the same.

### Background Art

One of the main tasks performed in pathology or the pathology department is to perform diagnosis by reading images of a patient's biological tissue and determining the condition, signs, or lesions of a specific disease.

This type of diagnosis relies on the experience and knowledge of skilled medical personnel over a long period of time. Recently, due to the development of machine learning, attempts are being made to automate tasks such as image recognition or classification using computer systems. In particular, attempts are being made to automate diagnosis previously performed by skilled medical personnel using artificial neural networks, a type of machine learning.

The biggest advantage that can be expected from the method of diagnosing biological images using an artificial neural network trained through a large amount of data is that it does not simply automate the experience and knowledge of conventionally skilled medical personnel, but rather finds characteristic elements through self-training and derives the desired answer, so that it is possible to find characteristics of disease factors in images that even skilled medical personnel were not aware of.

In general, in order to apply artificial neural networks to the diagnostic field of biological images, a skilled medical professional must annotate the state of a specific disease (e.g., whether cancer is present, lesion area caused by a specific disease, etc.) on a digital image scanned from a slide to create a large number of training data and then train an artificial neural network through this, wherein the training data used for training the artificial neural network are usually images of a single resolution.

Mastectomy, which removes the area with cancerous lesions, is widely used to treat breast cancer patients. In order to predict the postoperative prognosis of breast cancer patients or decide on additional anticancer treatment, pathological review of the resected breast cancer tissue is necessary, and it is a major pathological review item to distinguish the invasive cancer lesion area and the ductal carcinoma in situ (DCIS) lesion area in the breast cancer resection tissue and check the size, ratio and the like.

Sine traditional optical microscope-based visual reading and measurement of size, ratio, etc., not only increases the fatigue of the pathologist who is the reader, but also causes inaccuracy due to subjective reading, there is a great need to classify and detect lesion areas and measure their sizes through the application of artificial neural network-based image analysis deep learning technology.

In order to determine whether invasive cancer or DCIS is present in the breast cancer lesion area using an artificial neural network such as a convolutional neural network, a macroscopic shape of the tissue is very important, and at the same time, a microscopic cell shape and a size of the cell nucleus are also very important. However, the existing single-resolution convolutional neural network has limitations in capturing these characteristics well and accurately classifying the lesion area.

### Disclosure of the Invention

### Technical Goals

A technical object to be achieved by the present disclosure is to propose a structure of an artificial neural network that can detect lesion areas caused by a specific disease from biological tissue images and to provide a method for training the same. More specifically, an object of the present disclosure is to propose a structure of an artificial neural network that can determine the lesion area by considering both the tissue shape which is a macroscopic characteristic of the tissue area, and the microscopic characteristics, such as the shape of the cell or the size of the cell nucleus, and provide a method for training the same.

In particular, in the case of breast cancer, since there is a high need to consider both the macroscopic shape and microscopic characteristics of the tissue in determining whether the breast cancer lesion area is invasive cancer or DCIS, an object of the present disclosure is to propose a deep learning model for biological image analysis that detects lesion areas in breast cancer resection tissue by considering both the macroscopic tissue shape, microscopic cell shape, and cell nucleus size, and classifies the detected lesion area as invasive cancer or DCIS.

### Technical Solutions

According to one aspect of the present disclosure, there is provided an artificial neural network training method, including, acquiring, by an artificial neural network training system, a slide image of a biological tissue slide, acquiring, by the artificial neural network training system, a first high-resolution patch to an N-th high-resolution patch (where N is an integer of 2 or more) from the slide image, acquiring, by the artificial neural network training system, an i-th low-resolution patch corresponding to an i-th high-resolution patch (where i is an any integer of 1<=i<=N), wherein the i-th high-resolution patch and the corresponding i-th low-resolution patch have the same size, and a center point of the i-th high-resolution patch and a center point of the i-th low-resolution patch point to the same location on the biological tissue slide, and training, by the artificial neural network training system, an artificial neural network by inputting the i-th high-resolution patch and the i-th low-resolution patch, wherein the artificial neural network includes a first encoding convolutional neural network; a second encoding convolutional neural network; and a decoding convolutional neural network, and wherein the first encoding convolutional neural network is a convolutional neural network configured to receive the i-th high-resolution patch to output a first feature map corresponding to the i-th high-resolution patch, the second encoding convolutional neural network is a convolutional neural network configured to receive the i-th low-resolution patch to output context information corresponding to the i-th low-resolution patch, and the decoding convolutional neural network is a convolutional neural network configured to reflect the context information corresponding to the i-th low-resolution patch in the first feature map corresponding to the i-th high-resolution patch, and generate predetermined prediction information to determine a lesion area within the i-th high-resolution patch based on a result value reflecting the context information.

In an embodiment, the biological tissue slide may be a breast cancer resection tissue slide, and the slide image may be annotated with an invasive cancer area which is a lesion area caused by invasive cancer, and a ductal carcinoma in situ area which is a lesion area caused by ductal carcinoma in situ.

In an embodiment, the decoding convolutional neural network may include a first convolutional layer configured to perform a convolution operation on the first feature map, and a first post-processing layer configured to reflect the context information in the first feature map, by determining a normalization parameter using the context information output from the second encoding convolutional neural network, and performing adaptive normalization on a result value output from the first convolutional layer with the determined normalization parameter.

In an embodiment, the decoding convolutional neural network may include a first convolutional layer configured to perform a convolution operation on the first feature map, and a first post-processing layer configured to reflect the context information in the first feature map, by performing an attention mechanism based on the context information output from the second encoding convolutional neural network on a result value output from the first convolutional layer.

In an embodiment, the first encoding convolutional neural network may be configured to further output a second feature map corresponding to the i-th high-resolution patch, wherein the second feature map is a lower-level feature map than the first feature map, the decoding convolutional neural network may further include a non-local block layer configured to perform a non-local block operation on the second feature map, a concatenation layer configured to concatenate a result delivered from the first post-processing layer and a result delivered from the non-local block layer, a second convolutional layer configured to perform a convolution operation on a result delivered from the concatenation layer, and a second post-processing layer configured to reflect the context information corresponding to the i-th low-resolution patch in a result output from the second convolutional layer, and the decoding convolutional neural network may be configured to output the prediction information based on a result output from the second post-processing layer.

According to another aspect of the present disclosure, there is provided a method of providing a determination result for a predetermined determination target biological tissue slide through an artificial neural network trained by the artificial neural network training method, including, acquiring, by a computing system, a determination target slide image of the determination target biological tissue slide, generating, by the computing system, a first determination target high-resolution patch to an N-th determination target high-resolution patch from the determination target slide image, generating, by the computing system, a j-th determination target low-resolution patch corresponding to a j-th determination target high-resolution patch (where j is an any integer of 1< j<=N), wherein the j-th determination target high-resolution patch and the corresponding j-th determination target low-resolution patch have the same size, and a center point of the j-th determination target high-resolution patch and a center point of the j-th determination target low-resolution patch point to the same location on the determination target biological tissue slide, and determining, by the computing system, a lesion area included in the j-th determination target high-resolution patch based on a prediction result output by the artificial neural network which receives the j-th determination target high-resolution patch and the j-th determination target low-resolution patch.

According to another aspect of the present disclosure, there is provided a computer program recorded on a non-transitory medium installed in a data processing device and for performing the method as described above.

According to another aspect of the present disclosure, there is provided a non-transitory computer-readable recording medium on which a computer program for performing the method as described above.

According to another aspect of the present disclosure, there is provided an artificial neural network training system, including a processor, and a memory in which a computer program is stored, wherein the computer program is configured to, when executed by the processor, cause the artificial neural network training system to perform the artificial neural network training method.

According to another aspect of the present disclosure, there is provided a determination result providing system for a predetermined determination target biological tissue slide, including, a processor, and a memory in which a computer program is stored, wherein the computer program is configured to, when executed by the processor, cause the determination result providing system to perform the method of providing the determination result.

### Advantageous Effects

According to the technical idea of the present disclosure, it is possible to determine a lesion area by considering both the tissue shape, which is a macroscopic characteristic of a tissue area, and the cell shape or cell nucleus size, which is a microscopic characteristic. In other words, it is possible to provide an artificial neural network that determines the lesion area by considering microscopic characteristics of the tissue through high-resolution images and macroscopic characteristics of the tissue through low-resolution images at the same time, and a method for training the same.

In particular, in the case of breast cancer, since there is a high need to consider both the macroscopic shape and microscopic characteristics of the tissue in determining whether the breast cancer lesion area is invasive cancer or DCIS, when an artificial neural network and a method for training the same according to the technical idea of the present disclosure are applied to a slide image of breast cancer resection tissue, it is possible to detect a lesion area very effectively, and to classify the detected lesion area as invasive cancer or DCIS.

### Brief Description of Drawings

In order to more fully understand the drawings cited in the detailed description of the present disclosure, a brief description of each drawing is provided.
FIG. 1 is a diagram schematically illustrating an environment in which an artificial neural network training method and a determination result providing method for a biological tissue slide are performed in accordance with an embodiment of the present disclosure.
FIG. 2 is a flowchart for explaining an artificial neural network training method in accordance with an embodiment of the present disclosure.
FIG. 3 is a diagram illustrating an example of a biological tissue slide image in which an invasive cancer area and a DCIS area are annotated.
FIG. 4A is a diagram for explaining a high-resolution patch.
FIG. 4B is a diagram illustrating an example of an area covered by a high-resolution patch and an area covered by a corresponding low-resolution patch with the same location as a center point on a biological tissue slide image.
FIG. 5 is a diagram for explaining a structure of an artificial neural network in accordance with an embodiment of the present disclosure.
FIG. 6 is a flowchart illustrating an example of a determination result providing method for a biological tissue slide in accordance with an embodiment of the present disclosure.
FIG. 7 is a diagram illustrating a schematic configuration of an artificial neural network training system in accordance with an embodiment of the present disclosure, and FIG. 8 is a diagram illustrating a schematic configuration of a determination result providing system in accordance with an embodiment of the present disclosure.

### Best Mode for Carrying Out the Invention

Since the present disclosure may be modified variously and have various embodiments, specific embodiments will be illustrated in the drawings and described in detail in the detailed description. However, this is not intended to limit the present disclosure to specific embodiments, and it should be understood to include all modifications, equivalents, and substitutes included in the spirit and scope of the present disclosure. In describing the present invention, if it is determined that a detailed description of related known technologies may obscure the gist of the present disclosure, the detailed description will be omitted.

Terms such as first and second may be used to describe various components, but the components should not be limited by the terms. Terms such as first and second do not indicate a particular order and are used only for the purpose of distinguishing one component from another component.

The terms used in the present application are used only to describe a particular embodiment and are not intended to limit the present disclosure. Singular expressions include plural expressions unless the context clearly means otherwise.

In this specification, it should be understood that terms such as "include" or "have" are intended to designate the presence of features, numbers, steps, operations, components, parts, or combinations thereof described in the specification, but do not preclude the possibility of the presence or addition of one or more other features, numbers, steps, operations, components, parts, or combinations thereof.

Additionally, in this specification, when one component 'transmits' data to another component, this means that the component may transmit the data directly to the other component or transmit the data to the other component through at least one other component. Conversely, when one component 'directly transmits' data to another component, it means that the data is transmitted from the component to the other component without going through still other component.

Hereinafter, with reference to the accompanying drawings, the present disclosure will be described in detail centering on embodiments of the present disclosure. Like reference numerals in each drawing indicate like members.

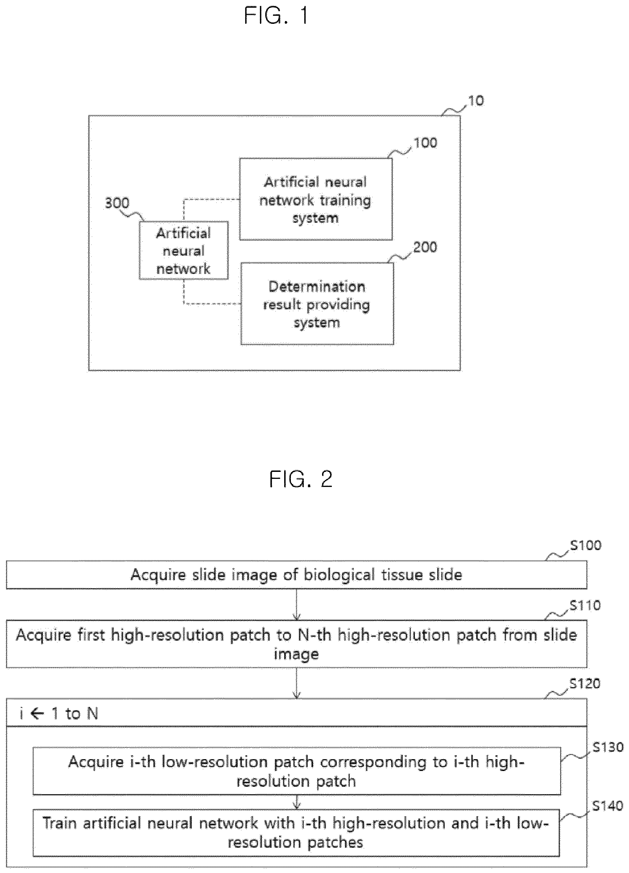

FIG. 1 is a diagram schematically illustrating an environment in which an artificial neural network training method and a determination result providing method for a biological tissue slide are performed in accordance with an embodiment of the present disclosure.

Referring to FIG. 1, the artificial neural network training method according to an embodiment of the present disclosure may be performed by an artificial neural network training system 100, andthe determination result providing method for a biological tissue slide according to an embodiment of the present disclosure may be performed by a determination result providing system 200 for a predetermined determination target biological tissue slide (hereinafter referred to as the 'determination result providing system').

The artificial neural network training system 100 may train an artificial neural network 300.

The artificial neural network training system 100 may train the artificial neural network 300 based on training data generated from a plurality of pathological specimens.

Pathological specimens may be biopsies collected from various organs of the human body or biological tissues excised through surgery. The artificial neural network training system 100 may generate individual training data using digital pathological slide images of pathological specimens, and input this to the input layer of the artificial neural network 300 to train the artificial neural network 300.

In an embodiment, the artificial neural network 300 may be an artificial neural network that may be trained to output a probability value for whether a given disease is expressed. The artificial neural network 300 may output a numerical value, i.e., a probability value, representing the determination result (e.g., whether the lesion is due to a specific disease (in particular, breast cancer)) for the target specimen based on data input through the input layer.

In the present specification, the artificial neural network is a neural network artificially constructed based on the operating principles of human neurons, includes a multilayer perceptron model, and may refer to a set of information expressing a series of design details that define the artificial neural network.

In an embodiment, the artificial neural network 300 may be a convolutional neural network or may include a convolutional neural network.

The convolutional neural network, as is well known, may include an input layer, a plurality of hidden layers, and an output layer. Each of the plurality of hidden layers may include a convolutional layer and a pooling layer (or a subsampling layer). The convolutional neural network may be defined by functions, filters, strides, weight factors, etc., to define each of these layers. In addition, the output layer may be defined as a fully connected feedforward layer. The design details for each layer that makes up a convolutional neural network are widely known. For example, known functions may be used for each of the convolutional function, pooling function, and activation function for defining the number of layers included in a plurality of layers and the plurality of layers, or separately defined functions may be used to implement the technical idea of the present disclosure.

Meanwhile, the trained artificial neural network 300 may be stored in the determination result providing system 200, and the determination result providing system 200 may use the trained artificial neural network 300 to make a determination about a predetermined diagnostic target specimen or a slide image of the diagnostic target specimen.

In an embodiment, the artificial neural network 300 may be a neural network that receives a biological tissue slide image or a patch (also referred to as a 'tile') which is part of a biological tissue slide image and provides diagnostic information, prognostic information, and/or response information to a treatment method for the biological tissue.

In particular, in an embodiment, the artificial neural network 300 may receive a biological tissue slide image or a patch which is part of a biological tissue slide image.

The slide image may be a scanned image of a biological tissue slide, and the patch may be a portion of the biological tissue slide image divided into a grid.

In an embodiment, the biological tissue slide may be a breast cancer resection tissue slide, and in this case, the lesion area may include an invasive cancer area, which is a lesion area due to invasive cancer, and a DCIS area, which is a lesion area due to DCIS.

Meanwhile, the artificial neural network 300 may be a region division model (i.e., pixel-level classification model) for the input image. In other words, the output value of the artificial neural network 300 may be a region division result (i.e., a pixel-level classification result). In other words, the artificial neural network 300 may output predetermined prediction information to determine the lesion area in the corresponding image. In addition, the artificial neural network 300 may be a pixel-level classification neural network that classifies the input image on a per-pixel level. For example, the artificial neural network 300 may be a neural network that outputs the probability of invasive cancer, the probability of DCIS, and the probability of not cancer for each pixel constituting the image.

The artificial neural network training system 100 may train the artificial neural network 300 by inputting a plurality of biological tissue patches. At this time, the training data (i.e., biological tissue patch) may be annotated with an invasive cancer area, which is a lesion area caused by invasive cancer, and a DCIS area, which is a lesion area caused by DCIS.

The determination result providing system 200 may make various determinations (e.g., determination on the lesion area, whether a disease is expressed, prognosis, determination on treatment method, etc.) about the target specimen using the trained artificial neural network 300.

The artificial neural network training system 100 and/or the determination result providing system 200 may be a computing system which is a data processing device with computing capabilities to implement the technical idea of the present disclosure, and in general, may include computing devices such as personal computers and mobile terminals as well as servers, which are data processing devices that may be accessed by clients through a network.

An average expert in the technical field of the present disclosure may easily infer that the artificial neural network training system 100 and/or the determination result providing system 200 may be implemented as a single physical device, but if necessary, a plurality of physical devices may be organically combined to implement the artificial neural network training system 100 and/or the determination result providing system 200 according to the technical idea of the present disclosure.

As shown in FIG. 1, the artificial neural network training system 100 and/or the determination result providing system 200 may be implemented in the form of a subsystem of a predetermined parent system 10. The parent system 10 may be a server. An average expert in the technical field of the present disclosure may easily infer that the server 10 refers to a data processing device with computing capabilities to implement the technical idea of the present disclosure, and in general, any device that may perform a specific service, such as a personal computer or mobile terminal, as well as a data processing device that may be accessed by a client through a network, may be defined as a server.

Alternatively, depending on the embodiment, the artificial neural network training system 100 and the determination result providing system 200 may be implemented separately from each other.

FIG. 2 is a flowchart for explaining an artificial neural network training method in accordance with an embodiment of the present disclosure.

Referring to FIG. 2, the artificial neural network training system 100 may acquire a biological tissue slide image (S 100).

On the biological tissue slide image, the lesion area may be annotated. In an embodiment, the biological tissue slide image may be annotated with an invasive cancer area, which is a lesion area caused by invasive cancer, and a DCIS area, which is a lesion area caused by DCIS.

Invasive cancer and DCIS may exist simultaneously in one biological tissue slide image, and the type of lesion may be annotated for each lesion area.

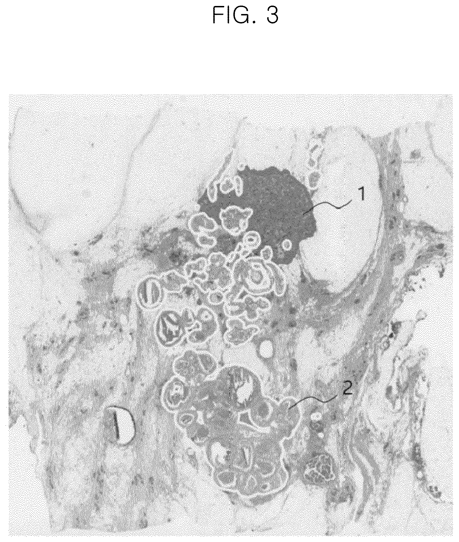

FIG. 3 is a diagram illustrating an example of a biological tissue slide image in which an invasive cancer area and a DCIS area are annotated. In FIG. 3, the area shown in red (e.g., 1) is the invasive cancer area, and the area shown in yellow (e.g., 2) is the DCIS area.

Referring again to FIG. 2, the artificial neural network training system 100 may acquire N high-resolution patches (where N is an integer of 2 or more) from the biological tissue slide image (S110). Here, high resolution does not mean a specific magnification or higher than a specific resolution, but rather means that it has a relatively high resolution compared to the low-resolution patch described later.

In an embodiment, the artificial neural network training system 100 may acquire the N high-resolution patches by dividing the biological tissue slide image into constant sizes.

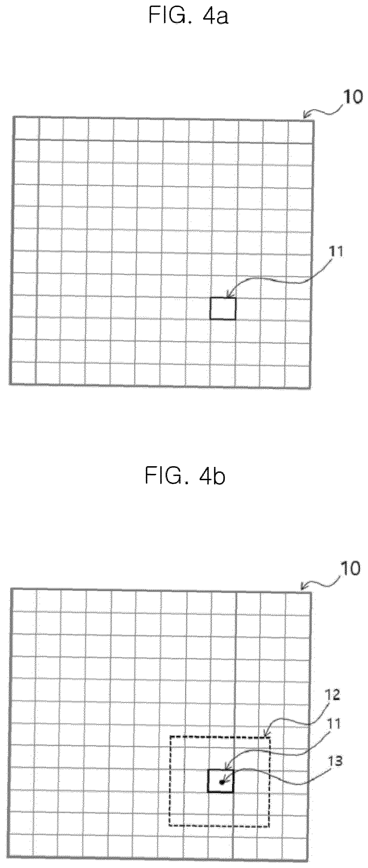

FIG. 4A is a diagram for explaining a high-resolution patch. Referring to FIG. 4A, the artificial neural network training system 100 may generate a high-resolution patch (e.g., 11) by dividing the biological tissue slide image 10 into a grid shape.

As in the example shown in FIG. 4A, when all of the N high-resolution patches are matched, an original biological tissue slide image may be created. The N high-resolution patches may be mutually exclusive, but are not limited thereto, and at least some of the N high-resolution patches may have areas that overlap with other high-resolution patches.

Meanwhile, the artificial neural network training system 100 may acquire the i-th low-resolution patch corresponding to the i-th high-resolution patch for all integers where 1<=i<=N (S120, S130).

The low-resolution patch may be a patch with a relatively lower resolution than the high-resolution patch. For example, if a high-resolution patch is a 50x magnification image, a low-resolution patch may be a 12.5x magnification image, and hereinafter, for ease of understanding, unless otherwise specified, it will be explained assuming that the high-resolution patch is a 50x magnification image and the low-resolution patch is a 12.5x magnification image.

The center point of the i-th high-resolution patch and the center point of the i-th low-resolution patch may point to the same location on the biological tissue slide. In addition, the i-th high-resolution patch and the i-th low-resolution patch may have the same size. For example, if the size of the high-resolution patch is 256×256, the size of the low-resolution patch may also be 256×256. Meanwhile, if the high-resolution patch is a 50x magnification image and the low-resolution patch is a 12.5x magnification image, which is 1/4 the ratio, the area of the area on the slide image covered by the high-resolution patch and the area of the area on the slide image covered by the corresponding low-resolution patch may be 1: 16.

In an embodiment, the artificial neural network training system 100 may extract a wide part (strictly the part covered by the low-resolution patch) around the center point of the high-resolution patch and then reduce it to acquire a low-resolution patch. For example, in order to acquire a low-resolution patch corresponding to a high-resolution patch whose size is 256 × 256 and the coordinates of the center point are (2048, 2560), the artificial neural network training system 100 may generate a low-resolution patch by extracting a 1024 × 1024 area centered on coordinates (2048, 2560) from the biological slide image and then reducing it to a size of 256 × 256.

FIG. 4B is a diagram illustrating an example of an area covered by a high-resolution patch and an area covered by a corresponding low-resolution patch with the same location as a center point on a biological tissue slide image. Referring to FIG. 4B, the center point of both the high-resolution patch 11 and the corresponding low-resolution patch may be the same location 13 on the biological tissue slide 10, and the area of the area 12 covered by the low-resolution patch may be 16 times the area of the area 11 covered by the high-resolution patch.

As in the example described above, the biological tissue slide may include only a single magnification image, but depending on the embodiment, the biological tissue slide may include multiple images from high magnification to low magnification in a pyramid format. For example, a biological tissue image may include a high-resolution slide image at 50x magnification and a low-resolution slide image at 12.5x magnification. In this case, the artificial neural network training system 100 may divide the high-resolution slide image to acquire a plurality of high-resolution patches, and for each high-resolution patch, the corresponding low-resolution patch may be extracted from the low-resolution slide image. For example, in order to acquire a low-resolution patch corresponding to a high-resolution patch whose size is 256 × 256 and the coordinates of the center point are (2048, 2560), the artificial neural network training system 100 may acquire a low-resolution patch by extracting a 256×256 area centered on coordinates (512, 640) from the low-resolution slide image.

Referring again to FIG. 2, the artificial neural network training system 100 may train the artificial neural network 300 by inputting the i-th high-resolution patch and the corresponding i-th low-resolution patch into the artificial neural network 300 (S140).

FIG. 2 shows the process of training the artificial neural network with a single biological tissue slide image, but in reality, the artificial neural network may be trained with multiple biological tissue slide images, and steps S100 to S140 of FIG. 2 may be performed for each biological tissue slide image.

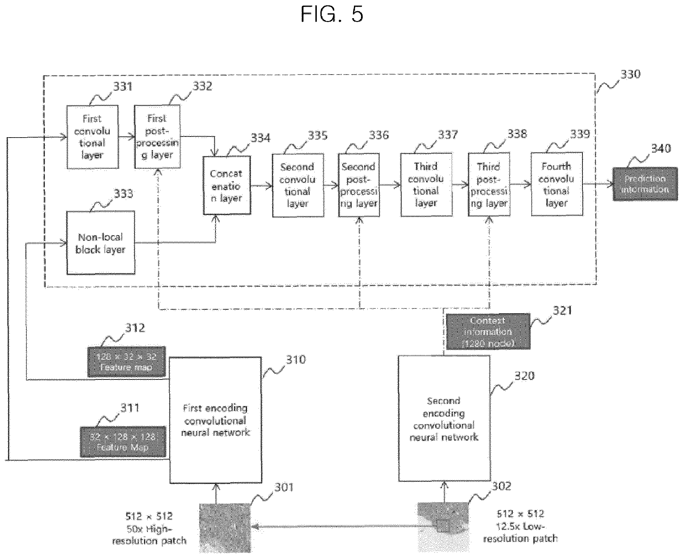

FIG. 5 is a diagram for explaining a structure of an artificial neural network 300 in accordance with an embodiment of the present disclosure.

Referring to FIG. 5, the artificial neural network 300 may include a first encoding convolutional neural network 310, a second encoding convolutional neural network 320, and a decoding convolutional neural network 330.

The first encoding convolutional neural network 310 and the second encoding convolutional neural network may be implemented as MNASNET, a type of convolutional neural network, but are not limited thereto, and may be implemented as other convolutional neural networks such as RESNET.

The first encoding convolutional neural network 310 may receive a high-resolution patch 301, and the second encoding convolutional neural network 320 may receive a low-resolution patch 302 corresponding to the high-resolution patch 301. In the example of FIG. 5, the first encoding convolutional neural network 310 receives a 50x high-resolution patch with a size of 512 × 512, and the second encoding convolutional neural network 320 receives a 12.5x low-resolution patch with a size of 512 × 512.

In addition, the first encoding convolutional neural network 310 may generate a feature map during the process of generating the final output. In other words, the feature map may be an intermediate product generated from the hidden layer (e.g., convolutional layer) of the first encoding convolutional neural network 310.

The first encoding convolutional neural network 310 may generate two or more feature maps corresponding to the input high-resolution patch 301, and FIG. 5 shows an example in which a first feature map 311, which is a low-level feature map, and a second feature map 312, which is a high-level feature map, are generated. The first feature map 311 may be a low-level feature map with dimensions of 32 × 128 × 128 (size 128 × 128, 32 channels), and the second feature map 312 may be a high-level feature map with dimensions of 128 × 32 × 32 (size 32 × 32, 128 channels). In the present specification, a low-level feature map may mean that it is generated in a hidden layer that is relatively close to the input layer compared to a high-level feature map, or that it is relatively less abstracted than a high-level feature map and has a larger amount of information. In addition, in the present specification, when the dimension of a value output from a specific neural network or layer is expressed as c × a × b, the corresponding value indicates that it is the value of the c channel whose size is a × b.

Meanwhile, the reason why a high-level feature map has a larger number of channels than a low-level feature map is that typically, the size of the image is reduced by half both horizontally and vertically through max pooling in the middle of the convolutional neural network, and at this time, the number of channels is increased to reduce information loss. In other words, while data flows from the convolutional layer close to the input to the convolutional layer close to the output, abstraction is attempted by reducing the size of the feature map, and instead, the number of channels is increased to increase the amount of abstract information.

Meanwhile, the second encoding convolutional neural network 320 may receive the low-resolution patch 302 and output context information 321 corresponding to the low-resolution patch 302.

The context information 321 output by the second encoding convolutional neural network 320 may not be the final output value of the second encoding convolutional neural network 320, and the output of the layer immediately preceding the fully connected layer for the final output of the second encoding convolutional neural network 320 may be context information 321.

FIG. 5 shows an example of outputting 1280-dimensional context information 321, but the size of the context information 321 may vary depending on the structure of the neural network.

Meanwhile, the decoding convolutional neural network 330 may reflect the context information 321 corresponding to the low-resolution patch 302 in the feature map 311 and/or 312 output by the first encoding convolutional neural network 310, and generate predetermined prediction information 337 for determining the lesion area within the high-resolution patch 301 based on a result value reflecting the context information 321.

In an embodiment, the decoding convolutional neural network 330 may output a probability that each pixel constituting the high-resolution patch 301 corresponds to a normal area/invasive cancer lesion area/DCIS lesion area. In this case, the decoding convolutional neural network 330 may output prediction information of 3 × 512 × 512 dimensions, but is not limited to this. Depending on the embodiment, the decoding convolutional neural network 330 may output a probability that each pixel group (e.g., a pixel group consisting of four square pixels) constituting the high-resolution patch 301 corresponds to a normal/invasive cancer lesion area/DCIS lesion area. In this case, the decoding convolutional neural network 330 may output prediction information with dimensions of 3 × 128 × 128.

The sum of the probability that each pixel or each pixel group is normal, the probability that it is an invasive cancer lesion area, and the probability that it is a DCIS lesion area may be 1.

Referring to FIG. 5, the decoding convolutional neural network 330 may include a first convolutional layer 331 and a first post-processing layer 332. The decoding convolutional neural network 330 may also further include a non-local block layer 333, a concatenation layer 334, a second convolutional layer 335, and a second post-processing layer 336.

Depending on the embodiment, the decoding convolutional neural network 330 may include only a portion of the layer shown in FIG. 5, or may further include other layers other than the layer shown in FIG. 5. For example, the decoding convolutional neural network 330 may include only the first convolutional layer 331 and the first post-processing layer 332 among the layers shown in FIG. 5, and in some cases, may further include one or more convolutional layers and post-processing layers.

The first convolutional layer 331 may perform a convolutional operation on the first feature map 311. For example, the first convolutional layer 331 may perform a convolutional operation through a 3 × 3 convolutional filter and output a result with dimensions of 32 × 128 × 128.

The first post-processing layer 332 may reflect the context information 321 output from the second encoding convolutional neural network 320 in the result generated by the first convolutional layer 331.

In an embodiment, the first post-processing layer 332 may reflect the context information 321 through an adaptive normalization technique. Adaptive normalization refers to a technique that has one fully connected layer that takes context information as input and outputs the average and standard deviation (or variance), which are normalization parameters, and performs normalization using the average and standard deviation output from this layer.

More specifically, the first post-processing layer 332 may, by determining normalization parameters (e.g., average and standard deviation) using the context information 321 and performing adaptive normalization on the result value output from the first convolutional layer 331 with the determined normalization parameters, reflect the context information 321 in the first feature map 311.

In another embodiment, the first post-processing layer 332 may reflect the context information 321 through an attention mechanism. In other words, the first post-processing layer 332 may, by performing an attention mechanism based on the context information 321 output from the second encoding convolutional neural network 320 on the result value output from the first convolutional layer 331, reflect the context information 321 in the first feature map 311.

In the attention mechanism, context information is used as input and passes through one fully connected layer that outputs parameters that may be used in the attention mechanism. There may be various ways to apply the attention mechanism. For example, there is a way to view the channel-level weight value (multiplying the weight values for each channel) as attention, and there may be a method of, in a self-attention structure such as a non-local block, generating the query part generated from the original input through a fully connected layer with only context information, or adjusting the size of the original input and context information by adjusting the size of the feature map, concatenating it, and then inputting it into a fully connected layer that generates a query.

Although not shown in FIG. 5, values output from post-processing layers (e.g., second post-processing layer 336) including the first post-processing layer 332, may be passed to the next layer through a predetermined activation function such as Relu or Sigmoid.

Meanwhile, the non-local block layer 333 may perform a non-local block operation on the second feature map. Non-local block operation refers to an operation used to calculate the non-local correlation of the input feature map, and a detailed explanation of this is disclosed in Kaiming He et al.'s paper "Non-local Neural Networks" (https://arxiv.org/pdf/1711.07971.pdf).

Meanwhile, although not shown in FIG. 5, upscaling may be performed on the value output from the non-local block layer 333, and through this, a result having the same size (e.g., 128 × 128) as the result output from the first post-processing layer 332 may be generated and delivered to the next layer (i.e., the concatenation layer 334). In addition, interpolation or transposed convolution may be used as an upscaling technique.

The concatenation layer 334 may concatenate the results delivered from the first post-processing layer 332 and the results delivered from the non-local block layer 333. The concatenation layer may perform concatenating operations through channel stacking. For example, when a result with dimensions of 32 × 128 × 128 is delivered from the first post-processing layer 332 and a result with dimensions of 128 × 128 × 128 is delivered from the non-local block layer 333, the concatenation layer 334 may output a concatenation result with dimensions of 160 × 128 × 128 through channel stacking.

The second convolutional layer 335 may perform a convolutional operation on the result output from the concatenation layer 334. For example, the second convolutional layer 334 may perform a convolutional operation through a 3 × 3 convolutional filter and output a result with dimensions of 128 × 128 × 128.

The second post-processing layer 336 may reflect context information 321 corresponding to the low-resolution patch in the result output from the second convolutional layer 335. The second post-processing layer 336 may also perform an adaptive normalization technique or attention mechanism applying the context information 321 like the first post-processing layer 335.

Meanwhile, the decoding convolutional neural network 330 may output the prediction information 340 based on the output result from the second post-processing layer 336.

According to an embodiment of the present disclosure, one or more additional convolutional layers and additional post-processing layers associated therewith may be further included in the middle of the second post-processing layer 336 and the layer finally outputting the prediction information 340. For example, as shown in FIG. 5, the decoding convolutional neural network 330 may further include a third convolutional layer 337 and a third post-processing layer 338. The additional convolutional layer performs a convolutional operation on the result value output from the previous layer, similar to the other convolutional layers described above, and the additional post-processing layer, like the other post-processing layers described above, may also perform an adaptive normalization technique or attention mechanism applying the context information 321.

In addition, depending on the embodiment, the decoding convolutional neural network 330 may output the prediction information 337 through additional layers (e.g., additional convolutional layer and/or fully connected layer, output layer, etc.). For example, as shown in FIG. 5, the decoding convolutional neural network 330 may further include a fourth convolutional layer 339. As described above, the artificial neural network 300 has a structure that reflects the value output from the encoding convolutional neural network that receives a low-resolution image including a large area of biological tissue in the output value of the encoding convolutional neural network which has a high-resolution image as input. In other words, the artificial neural network 300 has the advantage of being able to reflect all microscopic features extracted from high-resolution images that cover a narrow area but well represent detailed characteristics of the tissue as well as the macroscopic features of biological tissue that appear in low-resolution images covering a large area.

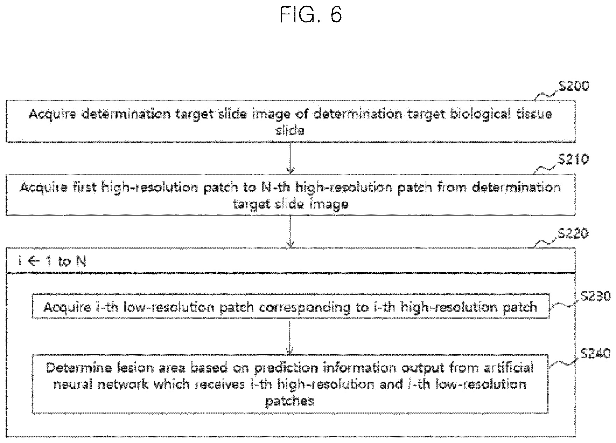

FIG. 6 is a flowchart illustrating an example of a determination result providing method for a biological tissue slide in accordance with an embodiment of the present disclosure. The determination result providing method for a biological tissue slide according to FIG. 6 may be performed by the determination result providing system 200, and in the determination result providing system 200, an artificial neural network 300 previously trained by the artificial neural network training system 100 may be stored.

Referring to FIG. 6, the determination result providing system 200 may acquire a determination target slide image which is a slide image of a predetermined determination target biological tissue slide (S200).

The determination result providing system 200 may acquire a first determination target high-resolution patch to an N-th determination target high-resolution patch from the determination target slide image (S220).

In addition, the determination result providing system 200 may generate low-resolution patches corresponding to the first determination target high-resolution patch to the N-th determination target high-resolution patch, respectively (S220, S230). At this time, the j-th determination target high-resolution patch and the j-th determination target low-resolution patch (j is an any integer where 1<=j<=N) have the same size, and the center point of the j-th high-resolution patch and the center point of the j-th low-resolution patch may point to the same location on the determination target biological tissue slide.

Since the process of acquiring the first determination target high-resolution patch to the N-th determination target high-resolution patch and a low-resolution patch corresponding to each of them from the determination target slide image is very similar to the process previously described with reference to FIGS. 4A to 4B, separate description will be omitted.

In addition, the determination result providing system 200 may determine the lesion area included in the j-th determination target high-resolution patch based on the prediction result output by the artificial neural network 300 that receives the j-th determination target high-resolution patch and the j-th determination target low-resolution patch (S240).

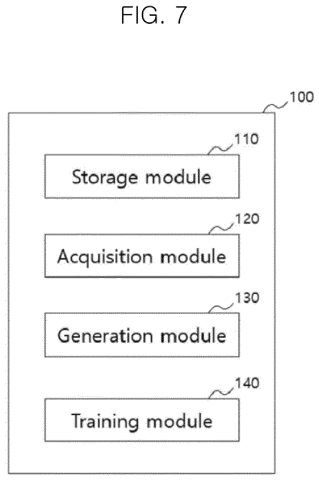

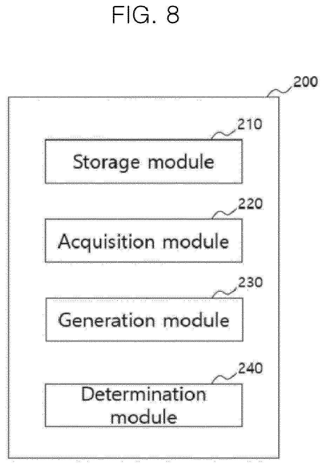

FIG. 7 is a diagram illustrating a schematic configuration of an artificial neural network training system 100 in accordance with an embodiment of the present disclosure, and FIG. 8 is a diagram illustrating a schematic configuration of a determination result providing system 200 in accordance with an embodiment of the present disclosure.

The artificial neural network training system 100 and the determination result providing system 200 may refer to a logical configuration provided with hardware resources and/or software necessary to implement the technical idea of the present disclosure, and does not necessarily mean one physical component or one device. In other words, the artificial neural network training system 100 and the determination result providing system 200 may refer to a logical combination of hardware and/or software provided to implement the technical idea of the present disclosure, and if necessary, it may be implemented as a set of logical components to implement the technical idea of the present disclosure by being installed in devices separated from each other and performing each function. In addition, the artificial neural network training system 100 and the determination result providing system 200 may refer to a set of configurations separately implemented for each function or role for implementing the technical idea of the present disclosure. Each configuration of the artificial neural network training system 100 and the determination result providing system 200 may be located on different physical devices or may be located on the same physical device. In addition, depending on the implementation example, the combination of software and/or hardware constituting each component of the artificial neural network training system 100 and the determination result providing system 200 is also located in different physical devices, and the components located in different physical devices may be organically combined to implement each of the modules.

In addition, the term module in the present specification may refer to a functional and structural combination of hardware for performing the technical idea of the present disclosure and software for driving the hardware. For example, the module may refer to a logical unit of a predetermined code and hardware resources for executing the predetermined code, and it may be easily inferred by an average expert in the technical field of the present disclosure that it does not necessarily mean a physically connected code or a single type of hardware.

Referring to FIG. 7, the artificial neural network training system 100 may include a storage module 110, an acquisition module 120, a generation module 130, and a training module 140. Depending on the embodiment of the present disclosure, some of the above-described components may not necessarily correspond to components essential for implementation of the present disclosure, and also, depending on the embodiment, the artificial neural network training system 100 may include more components than these. For example, the artificial neural network training system 100 may further include a communication module (not shown) for communicating with an external device, a control module (not shown) for controlling components and resources of the artificial neural network training system 100.

The storage module 110 may store an artificial neural network 30 to be trained. In addition, the storage module 110 may further store data (e.g., a biological tissue slide image annotated with a lesion area) to be used for training the artificial neural network 30.

The acquisition module 120 may acquire a slide image of a biological tissue slide.

The generation module 130 may generate a first high-resolution patch to an N-th high-resolution patch (where N is an integer of 2 or more) from the slide image, and acquire the i-th low-resolution patch corresponding to the i-th high-resolution patch (where i is an arbitrary integer where 1<=i<=N). Here, the i-th high-resolution patch and the corresponding i-th low-resolution patch have the same size, and the center point of the i-th high-resolution patch and the center point of the i-th low-resolution patch may point to the same location on the biological tissue slide.

The training module 140 may train the artificial neural network 300 by inputting the i-th high-resolution patch and the i-th low-resolution patch.

Referring to FIG. 8, the determination result providing system 200 may include a storage module 210, an acquisition module 220, a generation module 230, and a determination module 240. Depending on the embodiment of the present disclosure, some of the above-described components may not necessarily correspond to components essential for implementation of the present disclosure, and additionally, depending on the embodiment, the determination result providing system 200 may include more components than these. For example, the determination result providing system 200 may further include a communication module (not shown) for communicating with an external device, and a control module (not shown) for controlling components and resources of the determination result providing system 200.

The storage module 210 may store a pre-trained artificial neural network 300.

The acquisition module 220 may acquire a determination target slide image which is a slide image of a predetermined determination target biological tissue slide.

The generation module 230 may generate a first determination target high-resolution patch to an N-th determination target high-resolution patch from the determination target slide image, and generate the j-th determination target low-resolution patch corresponding to the j-th determination target high-resolution patch (where j is an arbitrary integer of 1<=j<=N). Here, the j-th determination target high-resolution patch and the corresponding j-th determination target low-resolution patch have the same size, and a center point of the j-th determination target high-resolution patch and a center point of the j-th determination target low-resolution patch point to the same location on the determination target biological tissue slide.

The determination module 240 may determine the lesion area included in the j-th determination target high-resolution patch, based on the prediction result output by the artificial neural network 300 that receives the j-th determination target high-resolution patch and the j-th determination target low-resolution patch.

Meanwhile, depending on the implementation example, the artificial neural network training system 100 and the determination result providing system 200 may include a processor and a memory that stores a program executed by the processor. The processor may include a single-core CPU or a multi-core CPU. The memory may include a high-speed random access memory and may include a non-volatile memory such as one or more magnetic disk storage devices, flash memory devices, or other non-volatile solid-state memory devices. Access to memory by the processor and other components may be controlled by a memory controller.

In addition, the method according to an embodiment of the present disclosure may be implemented in the form of a computer-readable program instruction and stored on a non-transitory computer-readable recording medium, and a control program and a target program according to an embodiment of the present disclosure may also be stored in a non-transitory computer-readable recording medium. A non-transitory computer-readable recording medium includes all types of recording devices in which data that may be read by a computer system is stored.

Program instructions recorded on the recording medium may be those specifically designed and configured for the present disclosure, or may be known and available to those skilled in the software field.

Examples of computer-readable recording medium include magnetic media such as hard disks, floppy disks, and magnetic tapes, optical media such as CD-ROM and DVD, magneto-optical media such as floptical disk, and hardware devices specifically configured to store and perform program instructions, such as ROM, RAM, flash memory, etc. In addition, the computer-readable recording medium may distributed in computer systems connected through a network, so that computer-readable codes may be stored and executed in a distributed manner.

Examples of program instructions include not only machine language code such as that created by a compiler, but also high-level language code that may be executed by a device that electronically processes information using an interpreter, for example, a computer.

The hardware devices described above may be configured to operate as one or more software modules to perform the operations of the present disclosure, and vice versa.

The description of the present disclosure described above is for illustrative purposes, and those skilled in the art will understand that the present disclosure may be easily modified into other specific forms without changing the technical idea or essential features of the present disclosure. Therefore, the embodiments described above should be understood in all respects as illustrative and not restrictive. For example, each component described as unitary may be implemented in a distributed manner, and similarly, components described as distributed may also be implemented in a combined form.

The scope of the present disclosure is indicated by the claims described below rather than the detailed description above, and all changes or modified forms derived from the meaning and scope of the claims and their equivalent concepts should be construed as being included in the scope of the present disclosure.

### Industrial Applicability

The present disclosure may be used in a training method for training an artificial neural network for determining a breast cancer lesion area, and a computing system for performing the same.

## Claims

1. An artificial neural network training method, comprising:
acquiring, by an artificial neural network training system, a slide image of a biological tissue slide;
acquiring, by the artificial neural network training system, a first high-resolution patch to an N-th high-resolution patch (where N is an integer of 2 or more) from the slide image;
acquiring, by the artificial neural network training system, an i-th low-resolution patch corresponding to an i-th high-resolution patch (where i is an any integer of 1<=i<=N), wherein the i-th high-resolution patch and the corresponding i-th low-resolution patch have the same size, and a center point of the i-th high-resolution patch and a center point of the i-th low-resolution patch point to the same location on the biological tissue slide; and
training, by the artificial neural network training system, an artificial neural network by inputting the i-th high-resolution patch and the i-th low-resolution patch,
wherein the artificial neural network comprises a first encoding convolutional neural network; a second encoding convolutional neural network; and a decoding convolutional neural network, and
wherein the first encoding convolutional neural network is a convolutional neural network configured to receive the i-th high-resolution patch to output a first feature map corresponding to the i-th high-resolution patch,
the second encoding convolutional neural network is a convolutional neural network configured to receive the i-th low-resolution patch to output context information corresponding to the i-th low-resolution patch, and
the decoding convolutional neural network is a convolutional neural network configured to reflect the context information corresponding to the i-th low-resolution patch in the first feature map corresponding to the i-th high-resolution patch, and generate predetermined prediction information to determine a lesion area within the i-th high-resolution patch based on a result value reflecting the context information.

2. The artificial neural network training method of claim 1, wherein the biological tissue slide is a breast cancer resection tissue slide, and
the slide image is annotated with an invasive cancer area which is a lesion area caused by invasive cancer, and a ductal carcinoma in situ area which is a lesion area caused by ductal carcinoma in situ.

3. The artificial neural network training method of claim 1, wherein the decoding convolutional neural network comprises:
a first convolutional layer configured to perform a convolution operation on the first feature map; and
a first post-processing layer configured to reflect the context information in the first feature map, by determining a normalization parameter using the context information output from the second encoding convolutional neural network, and performing adaptive normalization on a result value output from the first convolutional layer with the determined normalization parameter.

4. The artificial neural network training method of claim 1, wherein the decoding convolutional neural network comprises:
a first convolutional layer configured to perform a convolution operation on the first feature map; and
a first post-processing layer configured to reflect the context information in the first feature map, by performing an attention mechanism based on the context information output from the second encoding convolutional neural network on a result value output from the first convolutional layer.

5. The artificial neural network training method of claim 3 or 4, wherein the first encoding convolutional neural network is configured to further output a second feature map corresponding to the i-th high-resolution patch, wherein the second feature map is a lower-level feature map than the first feature map, and
wherein the decoding convolutional neural network further comprises:
a non-local block layer configured to perform a non-local block operation on the second feature map;
a concatenation layer configured to concatenate a result delivered from the first post-processing layer and a result delivered from the non-local block layer;
a second convolutional layer configured to perform a convolution operation on a result delivered from the concatenation layer; and
a second post-processing layer configured to reflect the context information corresponding to the i-th low-resolution patch in a result output from the second convolutional layer, and
the decoding convolutional neural network is configured to output the prediction information based on a result output from the second post-processing layer.

6. A method of providing a determination result for a predetermined determination target biological tissue slide through an artificial neural network trained by the artificial neural network training method of claim 1, the method comprising:
acquiring, by a computing system, a determination target slide image of the determination target biological tissue slide;
generating, by the computing system, a first determination target high-resolution patch to an N-th determination target high-resolution patch from the determination target slide image;
generating, by the computing system, a j-th determination target low-resolution patch corresponding to a j-th determination target high-resolution patch (where j is an any integer of 1<=j<=N), wherein the j-th determination target high-resolution patch and the corresponding j-th determination target low-resolution patch have the same size, and a center point of the j-th determination target high-resolution patch and a center point of the j-th determination target low-resolution patch point to the same location on the determination target biological tissue slide; and
determining, by the computing system, a lesion area included in the j-th determination target high-resolution patch based on a prediction result output by the artificial neural network which receives the j-th determination target high-resolution patch and the j-th determination target low-resolution patch.

7. A computer program installed in a data processing device and recorded on a non-transitory medium for performing the method of claim 1 or 6.

8. A non-transitory computer-readable recording medium on which a computer program for performing the method of claim 1 or 6 is recorded.

9. An artificial neural network training system, comprising:
a processor; and
a memory in which a computer program is stored,
wherein the computer program is configured to, when executed by the processor, cause the artificial neural network training system to perform an artificial neural network training method,
wherein the artificial neural network training method comprises:
acquiring, by the artificial neural network training system, a slide image of a biological tissue slide;
acquiring, by the artificial neural network training system, a first high-resolution patch to an N-th high-resolution patch (where N is an integer of 2 or more) from the slide image;
acquiring, by the artificial neural network training system, an i-th low-resolution patch corresponding to an i-th high-resolution patch (where i is an any integer of 1<=i<=N), wherein the i-th high-resolution patch and the corresponding i-th low-resolution patch have the same size, and a center point of the i-th high-resolution patch and a center point of the i-th low-resolution patch point to the same location on the biological tissue slide; and
training, by the artificial neural network training system, an artificial neural network by inputting the i-th high-resolution patch and the i-th low-resolution patch,
wherein the artificial neural network comprises a first encoding convolutional neural network; a second encoding convolutional neural network; and a decoding convolutional neural network, and
wherein the first encoding convolutional neural network is a convolutional neural network configured to receive the i-th high-resolution patch to output a first feature map corresponding to the i-th high-resolution patch,
the second encoding convolutional neural network is a convolutional neural network configured to receive the i-th low-resolution patch to output context information corresponding to the i-th low-resolution patch, and
the decoding convolutional neural network is a convolutional neural network configured to reflect the context information corresponding to the i-th low-resolution patch in the first feature map corresponding to the i-th high-resolution patch, and generate predetermined prediction information to determine a lesion area within the i-th high-resolution patch based on a result value reflecting the context information.

10. The artificial neural network training system of claim 9, wherein the biological tissue slide is a breast cancer resection tissue slide, and
the slide image is annotated with an invasive cancer area which is a lesion area caused by invasive cancer, and a ductal carcinoma in situ area which is a lesion area caused by ductal carcinoma in situ.

11. The artificial neural network training system of claim 9, wherein the decoding convolutional neural network comprises:
a first convolutional layer configured to perform a convolution operation on the first feature map; and
a first post-processing layer configured to reflect the context information in the first feature map, by determining a normalization parameter using the context information output from the second encoding convolutional neural network, and performing adaptive normalization on a result value output from the first convolutional layer with the determined normalization parameter.

12. The artificial neural network training system of claim 9, wherein the decoding convolutional neural network comprises:
a first convolutional layer configured to perform a convolution operation on the first feature map; and
a first post-processing layer configured to reflect the context information in the first feature map, by performing an attention mechanism based on the context information output from the second encoding convolutional neural network on a result value output from the first convolutional layer.

13. The artificial neural network training system of claim 11 or 12, wherein the first encoding convolutional neural network is configured to further output a second feature map corresponding to the i-th high-resolution patch, wherein the second feature map is a lower-level feature map than the first feature map, and
wherein the decoding convolutional neural network further comprises:
a non-local block layer configured to perform a non-local block operation on the second feature map;
a concatenation layer configured to concatenate a result delivered from the first post-processing layer and a result delivered from the non-local block layer;
a second convolutional layer configured to perform a convolution operation on a result delivered from the concatenation layer; and
a second post-processing layer configured to reflect the context information corresponding to the i-th low-resolution patch in a result output from the second convolutional layer, and
the decoding convolutional neural network is configured to output the prediction information based on a result output from the second post-processing layer.

14. A determination result providing system for a predetermined determination target biological tissue slide, comprising:
a processor; and
a memory in which a computer program is stored,
wherein the computer program is configured to, when executed by the processor, cause the determination result providing system to perform a method of providing a determination result for the determination target biological tissue slide through an artificial neural network trained by the artificial neural network training method of claim 1, and
wherein the method of providing the determination result comprises:
acquiring, by the determination result providing system, a determination target slide image of the determination target biological tissue slide;
generating, by the determination result providing system, a first determination target high-resolution patch to an N-th determination target high-resolution patch from the determination target slide image;
generating, by the determination result providing system, a j-th determination target low-resolution patch corresponding to a j-th determination target high-resolution patch (where j is an any integer of 1<=j<=N), wherein the j-th determination target high-resolution patch and the corresponding j-th determination target low-resolution patch have the same size, and a center point of the j-th determination target high-resolution patch and a center point of the j-th determination target low-resolution patch point to the same location on the determination target biological tissue slide; and
determining, by the determination result providing system, a lesion area included in the j-th determination target high-resolution patch based on a prediction result output by the artificial neural network which receives the j-th determination target high-resolution patch and the j-th determination target low-resolution patch.
